(19) Europäisches Patentamt
European Patent Office
Office européen des brevets

(11) Veröffentlichungsnummer: **0 295 598 A1**

# (12) EUROPÄISCHE PATENTANMELDUNG

(21) Anmeldenummer: **88109350.4**

(51) Int. Cl.4: **C07C 103/48**

(22) Anmeldetag: **11.06.88**

(30) Priorität: **19.06.87 DE 3720245**

(43) Veröffentlichungstag der Anmeldung: **21.12.88 Patentblatt 88/51**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Schmierer, Roland, Dr.**
**An der Weinleite 5a**
**D-8901 Todtenweis(DE)**
Erfinder: **Kunstmann, Rudolf, Dr.**
**Schlesierstrasse 9**
**D-8901 Aystetten(DE)**

## (54) Verfahren zur Herstellung von N-Alkoxycarbonylmethyl-N-formyl-aminen und -anilinen.

(57) Gegenstand der Erfindung ist ein Verfahren zur Herstellung von Verbindungen der Formel I,

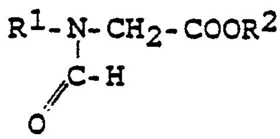

(I)

worin $R^1$ (subst.) Aryl oder (subst. ) Cyclohexyl und $R^2$ = Alkyl bedeutet, bei dem eine Verbindung der Formel $R^1-NH_2$ mit der mindest doppeltmolaren Menge von Glyoxylsäure in Gegenwart von Wasser umgesetzt wird und die erhaltene Säure anschließend verestert wird.

EP 0 295 598 A1

Xerox Copy Centre

## Verfahren zur Herstellung von N-Alkoxycarbonylmethyl-N-formyl-aminen und -anilinen

Gegenstand der vorliegenden Anmeldung ist ein Verfahren zur Herstellung von N-Alkoxycarbonylmethyl-N-formyl- aminen und -anilinen.

N-Alkoxycarbonylmethyl-N-formylamine bzw. -aniline sind wertvolle Zwischenprodukte von 1-substituierten-Imidazol-5-carbonsäurederivaten, die sowohl pharmakologische als auch biozide Wirksamkeit besitzen (s. z.B. DE-OS 2 732 531, DE-OS 3 217 094, DE-OS 3 444 918, DE-OS 3 514 116, US-PS 3 485 917, EP-A 0 207 563).

Die übliche Synthese dieser Verbindungen, wie sie z.B. in den Patentanmeldungen DE-OS 2 732 531, DE-OS 3 515 094 oder EP-A 207 563 beschrieben ist, führt entweder durch Alkylierung des Amins/Anilins mit Halogenessigsäureestern und anschließender N-Formylierung oder durch primäre N-Formylierung und anschließender Amidalkylierung mit Halogenessigestern zu den genannten Verbindungen.

Es wurde nun ein neues Verfahren gefunden, bei dem ein Amin bzw. Anilin mit wäßriger Glyoxylsäure umgesetzt wird und die erhaltene Säure anschließend verestert wird.

Gegenüber den bekannten Verfahren besitzt das neue Verfahren mehrere Vorteile:

- Es kann auf die Verwendung der reizenden Halogenessigesterderivate verzichtet werden.
- Es werden keine organischen oder anorganischen Hilfsbasen (Alkylierungsstufe der bekannten Verfahren) benötigt;
- Es entsteht nur Kohlendioxyd als Nebenprodukt.

Die Umsetzung von Aminen mit Glyoxylsäure und die anschließende Verseifung der Formylgruppe zu N-Alkylsubstituierten Glycinderivaten ist von J. Kihlberg et al. in Acta Chem. Scand. B 37 (1983) 911-6 beschrieben. Die Autoren setzen jedoch Glyoxylsäurehydrat als Reaktionskomponente ein und verweisen auf eine starke Hemmung der Reaktion in Gegenwart von Wasser. Überraschenderweise läßt sich jedoch das Verfahren mit der wesentlich billigeren wäßrigen Glyoxylsäure durchführen.

Die Erfindung bezieht sich also auf ein Verfahren zur Herstellung der Verbindungen der Formel I

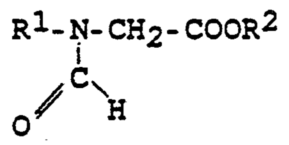

(I),

worin

$R^1$ einen Rest der Formeln

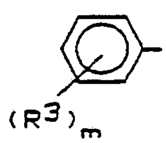 ; 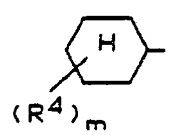 ; 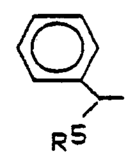 ;

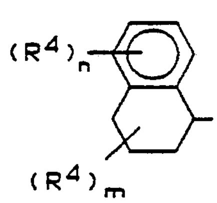 ; 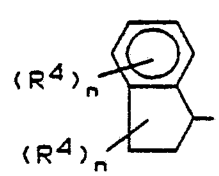 oder 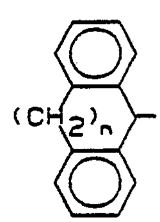

m 0, 1, 2, 3 oder 4,

n unabhängig voneinander 0, 1 oder 2,

$R^2$ $(C_1-C_6)$-Alkyl,

$R^3$ unabhängig voneinander $(C_1-C_3)$-Alkyl, Halogen oder $(C_1-C_4)$-Alkoxy;

$R^4$ unabhängig voneinander $(C_1-C_3)$-Alkyl und

$R^5$ Wasserstoff, $(C_1-C_4)$-Alkyl, Phenyl oder Cyclohexyl bedeutet,

dadurch gekennzeichnet, daß man a) ein Amin der Formel II mit der mindestens doppeltmolaren Menge

wäßriger

$R^1 - NH_2$ 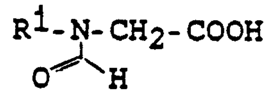

II III

Glyoxylsäure zur Carbonsäure der Formel III umsetzt und b) diese anschließend mit einem Alkohol der Formel $R^2OH$ verestert.

Für $R^1$ sind die folgenden Reste in Bezug auf das erfindungsgemäße Verfahren bevorzugt:

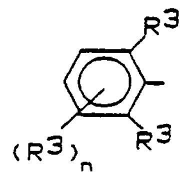 , 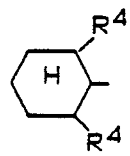 ,

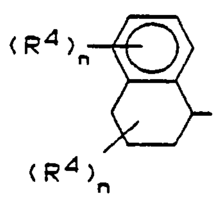 , 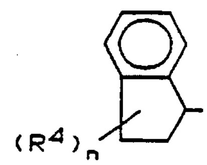 oder 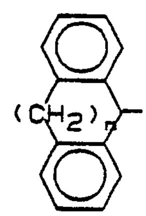

Unter Halogen wird vorzugsweise Chlor und Brom verstanden. Besitzen die Verbindungen der Formel I mindestens ein optisch aktives Zentrum, so bezieht sich das Herstellungsverfahren auf alle optischen Isomeren oder deren Gemische.

Für die Herstellung der als Zwischenstufe gebildeten Carbonsäure III wird, um einen vollständigen Umsatz des Amins bzw. Anilins zu erreichen, mindestens die 2,0 molare, vorzugsweise die 2,0 - 3,0 molare Menge Glyoxylsäure (bezogen auf II) benötigt. Der Wassergehalt der Glyoxylsäure kann zwischen 30 Gew.-% und 80 Gew-.% schwanken, wobei vorteilhafterweise ein technische Glyoxylsäurelösung (ca. 40 - 60 Gew.-%-Wasser) eingesetzt werden kann.

Die Temperaturen bei dem Verfahrensschritt a) können in Abhängigkeit vom verwendeten Lösungsmittel zwischen 0 °C und 150 °C variieren, wobei bevorzugt zwischen 20 °C und 120 °C gearbeitet wird. Das Fortschreiten der Reaktion ist leicht anhand der Gasentwicklung zu erkennen.

Der Verfahrensschrittt a) kann ohne oder vorteilhafter in Gegenwart eines organischen möglichst wassermischbaren Lösungsmittels wie z.B. Ethanol, Dimethylformamid, Ameisensäure besonders vorteilhaft in Gegenwart von Ameisensäure durchgeführt werden.

Die als Zwischenprodukt entstehende Säure der Formel III kann entweder isoliert z.B. durch Aufnehmen des Reaktionsgemischs in Wasser und Absaugen oder Extrahieren des Reaktionsprodukts oder durch Eindampfen des Lösungsmittels und Kristallisieren des Rückstands, oder aber - vorteilhaft nach Eindampfen des Lösungmittels - direkt mit einem Alkohol der Formel $R^2OH$ verestert werden.

Die Veresterung der Carbonsäure der Formel III kann nach üblichen Methoden (s. z.B. Organikum, VEB-Deutscher Verlag der Wissenschaften, Berlin 1973, Seiten 440 - 443) durchgeführt werden.

Die Ausbeuten nach Durchführung beider Reaktionsschritte a) und b) sind gut bis sehr gut und liegen in Abhängigkeit vom Amin bzw. Anilin bei ca. 60 - 95 % der Theorie.

Die folgenden Beispiele dienen zur weiteren Erläuterung der Erfindung.

## Herstellungsbeispiele

**Beispiel 1**

Methyl-2,6-diethyl-N-formyl-anilino-acetat

In 14,9 g (0,10 mol) 2,6-Diethylanilin und 100 ml Ameisensäure tropfte man bei Raumtemperatur unter leichter Erwärmung 32,6 g (0,22 mol) einer ca. 50 %igen wäßrigen Glyoxylsäurelösung zu. Man rührte unter starker Gasentwicklung 1 h bei 70° C, destillierte die Ameisensäure bis zu einer Innentemperatur von 120° C ab, ließ abkühlen, goß auf Wasser, saugte ab und trocknete den Feststoff. Man erhielt 21,8 g N-(2,6-Diethylphenyl)-N-formylglycin vom Schmelzpunkt 139-41° C.
Man nahm den Feststoff in 50 ml Methanol auf, gab 2 ml konzentrierte Schwefelsäure zu und erhitzte 5 Stunden unter Rückfluß. Nach dem Abkühlen werden das Reaktionsgemisch in Wasser aufgenommen und das Produkt mit Toluol extrahiert. Die organische Phase wurde eingedampft und der Rückstand im Hochvakuum destilliert. Man erhielt 19,5 g (78 % d. Th. bzgl. eingesetztem Anilin) Methyl-2,6-diethyl-N-formyl-anilino-acetat (Siedepunkt 126-9° C/0,5 mbar).

**Beispiel 2**

**Ethyl-2,6-diethyl-N-formyl-anilino-acetat**

74,5 g (0,50 mol) 2,6-Diethylanilin wurden mit 185 g (1,25 mol) einer ca. 50 %igen wäßrigen Glyoxylsäurelösung in 500 ml Ameisensäure analog zu Beispiel 1 umgesetzt. Nach dem Abdestillieren der Ameisensäure gab man 500 ml Chloroform, 200 ml Ethanol und 1 ml konzentrierte Schwefelsäure zu und destillierte bis zur Beendigung der Wasserbildung am Wasserabscheider. Man ließ abkühlen, dampfte ein und destillierte den Rückstand im Hochvakuum. Man erhielt 109 g Ethyl-2,6-diethyl-N-formyl-anilino-acetat (83 % d. Th.) als hellgelbes Öl vom Siedepunkt 145-7° C/0,5 mbar.

**Beispiel 3**

**Ethyl-N-formyl-2,6-diethylcyclohexylamino-acetat**

15,5 g (0,10 mol) 2,6-Diethylcyclohexylamin wurden mit 32,6 g (0,22 mol) einer 50 %igen wäßrigen Glyoxylsäurelösung in 100 ml Ameisensäure 2 Stunden auf 100° C erhitzt. Man destillierte bei leichtem Vakuum die Ameisensäure ab, goß den Rückstand in Wasser, saugte den ausgefallenen Feststoff ab und trocknete ihn. Man erhielt 18,9 g Säure vom Schmelkzpunkt 125-130° C. Zur Veresterung wurde 100 ml wasserfreies Ethanol zugegeben und unter Rückfluß 7 Stunden Chlorwasserstoffgas eingeleitet. Nach dem Abkühlen wurde eingedampft und der Rückstand über eine Kieselgelsäure chromatographiert (Laufmittel Petrolether (tiefsiedend) : Essigester = 7 : 3). Man erhielt 17,0 g (66 % der Theorie bzgl eingesetztem Amin) Ethyl-N-formyl-2,6-diethylcyclohexylamino-acetat, als farbloses Öl. Die Identifizierung erfolgte $^1H$-NMR-spektroskopisch.

**Beispiel 4**

**Ethyl-N-formyl-benzhydrylamino-acetat**

29,7 g (0,16 mol) Benzhydrylamin wurden mit 59,7 g (0,40 mol) einer 50 %igen Wäßrigen Glyoxylsäurelösung in 160 ml Ameisensäure 8 Stunden bei 100° C gerührt. Man destillierte die überschüssige Ameisensäure ab, ließ abkühlen, goß in Wasser, extrahierte mit Essigester, trocknete die organische Phase

über Natriumsulfat und dampfte ein. Nach der Zugabe von 200 ml absolutem Ethanol und 4,4 g konzentrierter Schwefelsäure rührte man 5 Stunden unter Rückfluß, ließ abkühlen, dampfte ein, nahm den Rückstand in 2-N-Natronlauge/Toluol auf, trocknete die organische Phase, dampfte ein und chromatographierte den Rückstand (Kieselgel; Laufmittel: Petrolether (triefsiedend) : Essigester = 8 : 2); Man erhielt 26,6 g (56 % d. Th.) Ethyl-N-formyl-benzhydrylamino-acetate, als farbloses Öl. Die Identifizierung erfolgte $^1$H-NMR-spektroskopisch.

**Beispiel 5b**

**Ethyl-N-formyl-1-tetralinylamino-acetat**

27,6 g (0,19 mol) 1-Aminotetralin wurden mit 70,3 g (0,48 mol) einer 50 %igen wäßrigen Glyoxylsäurelösung in 185 ml Ameisensäure 2 Stunden auf 150° C erhitzt. Man destillierte die überschüssige Ameisensäure ab, goß in Wasser und saugte den Feststoff ab (35,2 g Säure vom Fp. 135-140° C). Nach Zugabe von 150 ml wasserfreiem Ethanol und 2,6 g konzentrierter Schwefelsäure rührte man 6 Stunden unter Rückfluß, dampfte ein, nahm in 2-N-Natronlauge/Toluol auf, trocknete die Toluolphase mit Natriumsulfat, dampfte ein und chromatographierte den Rückstand (Kieselgel; Petrolether (tiefsiedend): Essigester = 7 : 3). Man erhielt 31,4 g (63 % d. Th.) Ethyl-N-formyl-1-tetralinylamino-acetat als hellgebes Öl. Die Identifizierung erfolgte $^1$H-NMR-spektroskopisch.

Weitere Beispiele sind in der nachfolgenden Tabelle 1 aufgeführt.

Tabelle 1 Glycinesterderivate der Formel I

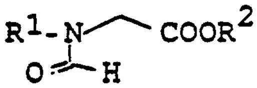

I

| Beisp. Nr. | $R^1$ | $R^2$ | $(R^3)_m$ |
|---|---|---|---|
| 6 | 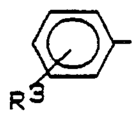 | $C_2H_5$ | 2,6-Dimethyl |
| 7 | " | " | 2-Ethyl-6-methyl |
| 8 | " | " | 2,6-Diethyl, 3-Cl |
| 9 | " | " | " , 3-Br |
| 10 | " | " | " , 3-Cl-4-methyl |
| 11 | " | n-$C_4H_9$ | " , 3,5-Dichlor |
| 12 | " | $C_2H_5$ | 2-Chlor-6-methyl, 4-Cl |
| 13 | " | " | 2-Butoxy-6-methyl |
| 14 | " | " | 2,6-Diisopropyl |
| 15 | 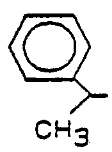 | $CH_3$ | - |
| 16 | 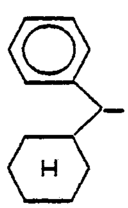 | $C_2H_5$ | - |

**Fortsetzung Tabelle 1**

| Beisp. Nr. | $R^1$ | $R^2$ | $(R^4)_m$ |
|---|---|---|---|
| 17 | 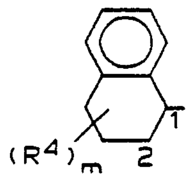 | $C_2H_5$ | 2-Methyl |
| 18 | " | " | 2,2-Dimethyl |
| 19 | " | " | 2-Ethyl |
| 20 | " | " | 2,4-Dimethyl |
| 21 | " | " | 2,2,4-Trimethyl |
| 22 | 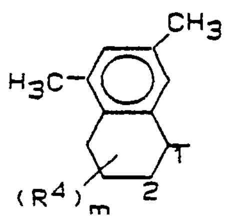 | " | H |
| 23 | " | " | 2,2-Dimethyl |
| 24 | 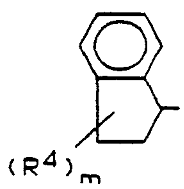 | " | H |
| 25 | " | " | 2-Methyl |
| 26 | " | " | 2-Ethyl |
| 27 | " | " | 2,2-Dimethyl |

**Fortsetzung Tabelle 1**

| Beisp. Nr. | $R^1$ | $R^2$ | $(R^4)_m$ |
|---|---|---|---|
| 28 | 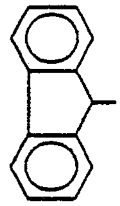 | $C_2H_5$ | - |
| 29 | 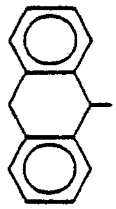 | " | - |

## Ansprüche

1. Verfahren zur Herstellung von Verbindungen der Formel I

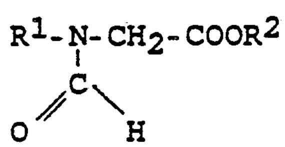

I

worin

$R^1$ einen Rest der Formeln

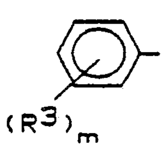 ; 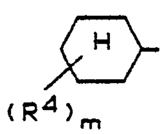 ; 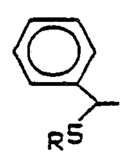 ;

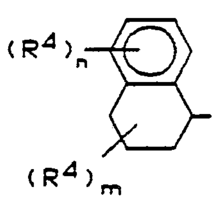 ; 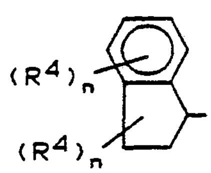 oder 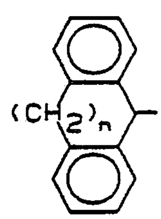

m 0, 1, 2, 3 oder 4,

n unabhängig voneinander 0, 1 oder 2,

$R^2$ $(C_1-C_6)$-Alkyl,

$R^3$ unabhängig voneinander $(C_1-C_3)$-Alkyl, Halogen oder $(C_1-C_4)$-Alkoxy;

$R^4$ unabhängig voneinander ($C_1$-$C_3$)-Alkyl und
$R^5$ Wasserstoff, ($C_1$-$C_4$)-Alkyl, Phenyl oder Cyclohexyl bedeutet,
dadurch gekennzeichnet, daß man a) ein Amin der Formel II mit der mindestens doppeltmolaren Menge wäßriger

$$R^1 - NH_2$$

II

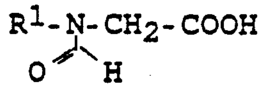

III

Glyoxylsäure gegebenenfalls in Gegenwart eines organischen Lösungsmittels zur Carbonsäure der Formel III umsetzt und b) diese anschließend mit einem Alkohol der Formel $R^2OH$ verestert.

2. Verfahren zur Herstellung von Verbindungen der Formel I gemäß Anspruch 1, dadurch gekennzeichnet, daß $R^1$ ein Rest der Formeln

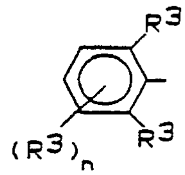 , 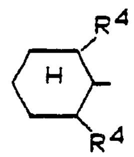 ,

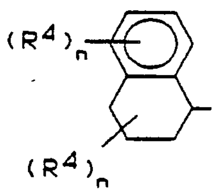 , 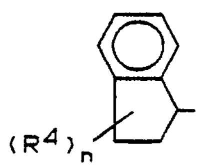 oder 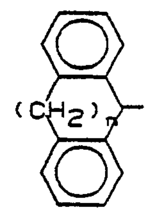

n unabhängig voneinander 0, 1 oder 2,
$R^2$ ($C_1$-$C_6$)-Alkyl,
$R^3$ unabhängig voneinander ($C_1$-$C_3$)-Alkyl, Halogen oder ($C_1$-$C_4$)-Alkoxy; und
$R^4$ unabhängig voneinander ($C_1$-$C_3$)-Alkyl bedeutet.

3. Verfahren gemäß Anspruch 1 oder 2, dadurch gekennzeichnet, daß man die 2,0 bis 3,0 molare Menge an Glyoxylsäure, bezogen auf die Komponente II in Form einer 20 bis 80 %igen wäßrigen Lösung einsetzt.

4. Verfahren gemäß einem oder mehreren der Ansprüche 1 bis 3, dadurch gekennzeichnet, daß man Glyoxylsäure in Form einer 40 bis 60 %igen Lösung einsetzt.

5. Verfahren gemäß einem oder mehreren der Ansprüche 1 bis 4, dadurch gekennzeichnet, daß die Reaktionstemperatur beim Verfahrensschritt a) 0 °C bis 150 °C beträgt.

6. Verfahren gemäß oder mehreren der Ansprüche 1 bis 5, dadurch gekennzeichnet, daß die Reaktionstemperatur bei dem Verfahrensschritt a) 20 °C bis 120 °C beträgt.

7. Verfahren gemäß einem oder mehreren der Ansprüche 1 bis 6, dadurch gekennzeichnet, daß beim Verfahrensschritt a) Ameisensäure als Lösungsmittel eingesetzt wird.

Europäisches Patentamt

# EUROPÄISCHER RECHERCHENBERICHT

Nummer der Anmeldung

EP 88 10 9350

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | ACTA CHEMICA SCANDINAVICA, Band B73, Seiten 911-916, Munksgaard, 1983 Copenhagen, DK; J. KIHLBERG et al.: "Synthesis of strombine. A new methd for monocarboxymethylation of primary amines" * ganzes Dokument * --- | 1-7 | C 07 C 103/48 |
| A | BERICHTE DER DEUTSCHEN CHEMISCHEN GESELLSCHAFT, Band 36(II), 1903, Seiten 2525-2526; E. ERLENMEYER JUN.: "Über die Einwirkung von Ammoniak auf ein Gemisch zweier Alpha-Oxosäuren." * Seite 2525 * ----- | 1 | |
| | | | RECHERCHIERTE SACHGEBIETE (Int. Cl.4) |
| | | | C 07 C 103/00 |

Der vorliegende Recherchenbericht wurde für alle Patentansprüche erstellt

| Recherchenort | Abschlußdatum der Recherche | Prüfer |
|---|---|---|
| BERLIN | 11-08-1988 | RUFET J.M.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)